# EUROPEAN PATENT APPLICATION

(11) **EP 4 813 515 A1**
(43) Date of publication of application: **30.09.2026**
(21) Application number: 25166837.2
(22) Date of filing: 27.03.2025
(51) Int. Cl.: B01J 35/70, B01J 35/80, B01J 37/00, B01J 37/03, C07C 31/04, B01J 35/61

(54) **METHANOL CATALYST WITH IMPROVED ACTIVITY AND STABILITY**

(71) Applicant: CLARIANT INTERNATIONAL LTD, 4132 Muttenz (CH)
(72) Inventor: Dybbert, Valentin, 83042 Rosenheim (DE); Zabilskiy, Maxim, 83024 Rosenheim (DE); Reitmeier, Stephan J., 85614 Kirchseeon (DE); Metzler, Martin, 83043 Bad Aibling (DE)
(74) Representative: Zusammenschluss Clariant

(57) **Abstract**

The present invention relates to a methanol catalyst with improved activity and stability, its preparation and a process for methanol synthesis by use of such catalyst.

## Description

### BACKGROUND OF THE INVENTION

The present invention relates to a methanol catalyst with improved activity and stability, its preparation and a process for methanol synthesis by use of such catalyst.

Methanol is an important chemical compound for the production of fine chemical and also as energy carrier. Mainly it is commercially obtained by the conversion of synthesis gas by the use of Cu based heterogenous catalysts.

DE102016225171 discloses an improved catalyst based on a tableted molded catalyst body, containing a metal-containing mixture, containing copper, zinc, and aluminum, with calcium aluminate as a binder material with a weight fraction of calcium aluminate in the range of 1.0% to 30.0%, for synthesizing methanol from synthesis gas. The catalyst is characterized by improved mechanical fracture strength and lateral crush strength, in particular in the reduced state.

EP 2 492 008 A1 concerns a catalyst for methanol synthesis with improved activity, lifetime and strength. It comprises essential components of copper, zinc and alumina, with a Cu/Zn ratio in the range of 1 to 3, an alumina content in the range of 3 to 20 % by weight, whereby alumina hydrate having a pseudo boehmite structure is utilized as alumina source.

In WO 2013/077835 A1 a catalyst for the dehydrogenation of alcohols is described, which contains highly dispersed Cu by reaction of an alumina formed by peptizing of boehmite or pseudoboehmite and precursors of ZrO2, ZnO and CuO.

WO 2021/099225 A1 relates to an improved chromium-free Cu-Al catalyst for the hydrogenation of carbonyl groups in organic compounds, characterized in that the catalyst contains zirconium in a proportion of 0.5 to 30.0 wt.%.

EP 0 482 753 A2 discloses a method of forming a methanol synthesis catalyst precursor comprises forming a precipitate comprising compounds, thermally decomposable to oxides or mixed oxides, of copper, zinc, aluminium and at least one element of Group IVB and/or Group VIIB of the Periodic Table of Elements.

CN 117816180 A relates to the preparation of a methanol catalyst by precipitating a Zn-Zr-Al compound, which is added to a Cu-Zn-Al containing solution, from which a Cu-Zn-Al compound is precipitated.

US 5,254,520 A describes a method of forming a methanol synthesis catalyst precursor comprises forming a precipitate comprising compounds, thermally decomposable to oxides or mixed oxides, of copper, zinc, aluminium and at least one element of Group IVB and/or Group VIIB of the Periodic Table of Elements.

There is still a demand for improved catalysts for methanol production, particularly for catalysts exhibiting enhanced catalytic activity, especially in a methanol process utilizing an CO₂ enriched feed, combined with increased mechanical stability in both oxidic and reduced states, allowing an improved loading of the catalyst into the reactor and preventing increased pressure drop during operation.

The underlying task of the invention was to develop a methanol catalyst with improved activity and stability, which can be utilized in a methanol process, especially a methanol process by utilizing an CO₂ enriched feed.

This task is solved by the inventive shaped catalyst body for methanol synthesis containing Cu, Zn and Al, which is characterized by weight percentages of graphite from 2.0 to 5.0 %, based on the total weight of the shaped catalyst body after loss on drying, and of zirconium, calculated as ZrO₂, from 1 to 10 %, based on the total weight of the catalyst after loss on ignition.

The weight percentage of graphite in the shaped catalyst body is in the range from 2.0 to 5.0 %, preferably 2.0 to 4.5 %, more preferably 2.0 to 4.0 %, even more preferably 3.0 to 4.0 %, most preferably 3.1 to 4.0 %, based on the total weight of the shaped catalyst body after loss on drying. In one embodiment the lower limit is 2.0, preferably 3.0, more preferably 3.1. In another embodiment the upper limit is 5.0, preferably 4.5, more preferably 4.0.

In one embodiment the graphite in the shaped catalyst body has a particle diameter D90 in the range from 2.0 µm to 17.5 µm, in particular in the range from 4.0 µm to 17.5 µm, preferably in the range from 5.0 µm to 15.0 µm and very particularly preferably in the range from 6.0 µm to 10.0 µm.

In a preferred embodiment, the graphite has a particle diameter D10 in the range from 1.0 µm to 4.0 µm and preferably in the range from 1.1 µm to 3.2 µm.

In a further embodiment, the graphite has a particle diameter D50 in the range from 2.0 µm to 9.0 µm and preferably in the range from 2.5 µm to 8.0 µm.

The shaped catalyst body according to the invention is further characterized by a zirconium content, calculated as ZrO₂, of from 1 to 10 % by weight, preferably from 2 to 9 % by weight, more preferably from 3 to 8 % by weight, most preferably from 4 to 7 % by weight, based on the total weight of the shaped catalyst body after loss on ignition. In one embodiment the lower limit of the zirconium content is 1 % by weight, preferably 2 % by weight, more preferably 3 % by weight, even more preferably 4 % by weight. In another embodiment the upper limit of the zirconium content is 10 % by weight, preferably 9 % by weight, more preferably 8 % by weight. The zirconium typically is present in its oxidic form and is X-ray amorphous. In the context of this invention X-ray amorphous means any solid zirconium containing compound form whose powder XRD pattern contains no Bragg reflections associated to any crystalline ZrO₂ phases.

The shaped catalyst body contains copper, zinc and aluminum, which are typically present in oxidic form. The Cu/Zn atomic ratio in the shaped catalyst body can vary within wide limits, but is preferably matched to that of conventional methanol synthesis catalysts. The Cu/Zn atomic ratio in the shaped catalyst body is preferably from 15:85 to 85:15, particularly preferably from 60:40 to 75:25. The Zn/Al atomic ratio is preferably from 60:40 to 80:20, particularly preferably from 70:30 to 80:20.

In one embodiment, the weight percentage of copper, calculated as CuO, is in the range from 40 to 70 %, preferably from 50 to 65 %, more preferably from 60 to 65 %, the weight percentage of zinc, calculated as ZnO, is in the range from 17 to 35 %, preferably from 20 to 30 %, more preferably from 25 to 30 %, and the weight percentage of aluminum, calculated as Al₂O₃, is in the range from 1 to 20 %, preferably from 1 to 18 %, more preferably from 1 to 10 %, even more preferably from 2 to 8 %, most preferably from 3 to 6 %, all based on the total weight of the shaped catalyst body after loss on ignition.

In one embodiment the catalyst does not contain any element of Group 5 to7 of the Period Table of Elements according to IUPAC notation (Pure and Appl. Chem., 1988, Vol. 60, No. 3, pp 431-436).

The catalyst according to the invention is present as a shaped catalyst body, in particular pressed as molded body, such as pressed tablet or pressed ring, pellet or extrudate. The shaped catalyst body can have various geometries, such as spheres, cylinders or hollow bodies such as rings. Spherical pellets can have a diameter d of 1 mm to 12 mm, preferably from 2 mm to 10 mm and particularly preferably from 3 mm to 8 mm.

If the shaped catalyst body is present in tableted form, it can have various dimensions. In one embodiment the diameter of the tablets is in the range from 2 to 8 mm and preferably from 3 to 7 mm. The diameter is particularly preferably in the range from 4.7 to 6.2 mm. In one embodiment the height of the tablets is in the range from 2 to 6 mm and preferably from 2.5 to 5 mm. The height is particularly preferably in the range from 2.8 to 4.2 mm.

If the shaped catalyst body is present in tableted form and in its oxidic form, it has an average side crush strength of from 80 to 300 N. In one embodiment it has an average side crush strength of from160 to 250 N. In another embodiment it has an average side crush strength of from 80 to 115 N.

In one embodiment the average side crush strength of the shaped catalyst body in tableted form based on the cross-sectional area is in the range from 5 to 12 N/mm².

In one embodiment the average side crush strength per gram of the shaped catalyst body in tableted form is in the range from 650 to 1300 N/g.

The shaped catalyst body typically comprises Cu, Zn and Al in oxidic form and can be further reduced to convert it in its reduced form, where at least part of the elemental components reveals oxidation state 0.

If the shaped catalyst body is present in tableted form and in its reduced form, it has an average side crush strength from 20 to 100 N, preferably 20 to 80 N, more preferably 30 to 60 N, even more preferably 35 to 60 N. Reduction of the shaped catalyst body can be carried out according to the procedure described in the subsequent experimental part of this application.

In one embodiment the shaped catalyst body has a BET surface area of from 75 to 150 m²/g, preferably from 80 to 140 m²/g, more preferably from 90 to 140 m²/g, even more preferably from 90 to 130 m²/g.

In one embodiment the shaped catalyst body is characterized by a content of fines with diameter below 1.7 mm of less than 3.0 wt.%, preferably less than 2.5 wt.%, more preferably less than 2.0 wt.%, determined by the drop test described in the experimental section.

The shaped catalyst body according to the invention is characterized by an LOI value less than 12 wt.%, preferably less than 10 wt.%.

Another subject of the invention is a method to prepare the inventive shaped catalyst body. Such method comprises the following steps:
a) combining (i) at least one aqueous medium A comprising one or more copper compounds, one or more zinc compounds and optionally one or more further transition metal compounds and (ii) at least one aqueous alkaline medium B to form a precipitate, wherein one/or more dissolved and/or dispersed aluminum compound and one or more zirconium compound is present in medium A and/or medium B and/or introduced to the mixture of medium A and B,
b) separating off the precipitate, optionally washing the precipitate,
c) drying the precipitate to obtain a dried precipitate,
d) calcining the dried precipitate from step c) at a temperature of between 300 and 800°C for a period of between 15 min and 4 h
e) shaping a mixture of the calcined precipitate from step d) and graphite to obtain a shaped catalyst body.

Suitable starting compounds for the compounds of copper, zinc, zirconium, and optional transition metal that are employed in step a) are in principle all compounds that are soluble in water or in basic or acidic aqueous solutions. Preference is given to using carbonates, nitrates, oxides, sulfates, acetates or formates.

The one or more aluminum compound can either already be present in the copper- and zinc-containing medium A or it can be added together with the precipitant in the form of the aqueous alkaline medium B or can be added directly to the combined mixture of medium A and B.

In one embodiment the one or more aluminum compound employed in step a) is soluble in water or in basic or acidic aqueous solutions and is present in its dissolved form in aqueous medium A or B in step a). In another embodiment the one or more aluminum compound is present in its dispersed form in the aqueous medium A or B, or is added to the mixture obtained after combining the aqueous medium A and B in step a).

The proportion of the one or more zirconium compound in step a) is chosen such that the proportion of zirconium, calculated as ZrO₂, in the shaped catalyst body is within a range from 1% to 10% by weight, preferably from 2 to 9 % by weight, more preferably from 3 to 8 % by weight, most preferably from 4 to 7 % by weight based on the total weight of the shaped catalyst body after loss on ignition.

The at least one aqueous medium A of one or more copper compounds, one or more zinc compounds and optionally one or more further transition metal compounds may be provided in the form of multiple separate aqueous solutions comprising one or more copper compounds, one or more zinc compounds, and optionally one or more further transition metal compounds, it being possible for any of these solutions to include an aluminum compound. Furthermore, one or more aqueous solutions of copper compounds, one or more aqueous solutions of zinc compounds, one or more aqueous solutions of aluminum compounds, and optionally one or more aqueous solutions of further transition metal compounds may be provided. These separate aqueous media can then be combined to an aqueous medium A comprising one or more copper compounds, one or more zinc compounds and optionally one or more further transition metal compounds.

In one embodiment, the at least one aqueous medium A comprising one or more copper compounds, one or more zinc compounds, and optionally one or more further transition metal compounds is, before being combined with the aqueous alkaline medium B, heated to a temperature above 20°C, for example a temperature within a range from 50°C to 90°C, preferably from 60 to 80°C, more preferably from 60 to 73°C, while preferably being stirred.

In a further embodiment, the aqueous alkaline medium B is, before being combined, with the at least one aqueous medium A heated to a temperature above 20°C, for example a temperature within a range from 50°C to 90°C, preferably from 60 to 80°C, more preferably from 60 to 73°C, while preferably being stirred.

In a further embodiment, the at least one aqueous medium A of one or more copper compounds, one or more zinc compounds, and optionally one or more further transition metal compounds and the aqueous alkaline medium B are both heated to a temperature within a range from 50°C to 90°C, preferably from 60 to 80°C, more preferably from 60 to 73°C, while preferably being stirred.

In one embodiment in step a) in addition to the addition of the one or more zirconium compound a further compound of a metal with oxidation state +4, preferably a titanium compound or hafnium compound, more preferably a hafnium compound, is added to the one or more aqueous media A or medium B or directly to the combined mixture of medium A and medium B. Suitable starting compounds for this compound of a metal with oxidation state +4 that are employed in step a) are in principle all compounds that are soluble in water or in basic or acidic aqueous solutions. Preference is given to using carbonates, nitrates, oxides, sulfates, acetates or formates.

The proportion of the compound of a metal with oxidation state +4 other than zirconium compound, preferably titanium compound or hafnium compound, more preferably hafnium compound, in step a) in medium A or medium B or in the mixture of medium A and B is chosen such that the proportion of the metal with oxidation state +4 other than zirconium compound in the final shaped catalyst body, calculated as its oxide, e.g. as TiO₂ or HfO₂, is within a range from 0.05 % to 5 % by weight, preferably from 0.08 to 4.0 % by weight, more preferably from 0.1 to 4.0 % by weight, based on the total weight of the shaped catalyst body after loss on ignition.

In one embodiment the shaped catalyst body according to the invention comprises either Na in a proportion by weight below 1%, or K in a proportion by weight below 1%, or Cs in a proportion by weight below 1%, or Fe in a proportion by weight below 0.1%, or Ni in a proportion by weight below 0.1%, or Cr in a proportion by weight below 0.1%, or Mn in a proportion by weight below 0.1%, or Ca in a proportion by weight below 5%, or Mg in a proportion by weight below 5%, or Bi in a proportion by weight below 0.1%, or Cl in a proportion by weight below 1%, or S in a proportion by weight below 0.1%, or As in a proportion by weight below 0.1% or any combination thereof, based on the total weight of the shaped catalyst body after loss on ignition.

In one embodiment, the precipitate in step a) is formed by passing the aqueous medium B comprising the precipitant into aqueous medium A comprising the dissolved compounds of copper, zinc, optional transition metal, and dissolved or dispersed aluminum compound preferably with constant stirring of the metal-containing solution.

In a further embodiment, the precipitate in step a) is formed by passing the aqueous alkaline solution B comprising the precipitant and the dissolved or dispersed aluminum compound into solution A comprising the dissolved compounds of copper, zinc, and optional transition metal, preferably with constant stirring of the metal-containing solution.

In a further embodiment, the aqueous alkaline medium B comprising the precipitant and the metal-containing medium A, wherein medium A and/or medium B additionally include a dissolved or dispersed aluminum compound, are jointly fed into a common precipitation vessel.

The temperature of the mixture in step a) after combination of medium A and B is usually within a range from 10 to 90°C, preferably between 50 and 90°C, more preferably within a range from 60 to 80°C, even more preferably from 60 to 73°C.

The pH during the precipitation of the metal-containing compounds in step a) is within a range from 6.0 to 8.0, preferably within a range from 6.0 to 7.5, more preferably within a range from 6.0 to 7.0.

After the precipitation, the resulting precipitate is separated off. This is typically done by filtration. Alternatively, the precipitate can also be separated off by decanting or centrifuging.

The separated precipitate can then optionally be subjected to one or more washing steps in order to remove any adhering impurities such as excess hydroxide ions or alkali metal ions. The precipitate can either remain directly in the filter chamber in the form of a filter cake and have a wash medium, preferably deionized water, passed through it, or it can alternatively be slurried in the wash medium and separated off again by means of a filter press, decantation or centrifugation. This process is usually repeated until the conductivity of the wash medium falls below a certain value. This is typically below 0.5 mS/cm, in particular below 0.2 mS/cm.

After being separated off and optionally washed, the precipitate is dried. In one embodiment drying takes place at a temperature within a range from 50 to 150°C, preferably within a range from 70 to 130°C, more preferably within a range from 80 to 120°C. The drying can take place in a spray dryer. Alternatively, drying can also take place in a stationary oven, in which case the drying time is usually within a range from 30 minutes to 6 h.

The dried powder is then subjected to calcination. This takes place at a temperature of between 300 and 800°C, preferably between 350 and 700°C, more preferably between 350 and 600°C, even more preferably between 400 and 600 °C. The duration of the calcination is between 15 minutes and 4 h, preferably between 15 minutes and 2 h.

In one embodiment the precipitation in step a) is controlled in such a way, that the distribution of at least Cu and Zn throughout the precipitate material is substantially homogenous.

The dried and calcined precipitate is then subjected to a shaping process. For this, the calcined precipitate obtained from step d) is subjected to the following step:
e) shaping a mixture of the calcined precipitate from step d) and graphite to obtain a shaped catalyst body.

Customary shaping processes are tableting, extrusion, and pelletization. In a preferred embodiment, the calcined precipitate is tableted.

Tableting is usually carried out with a tablet press such as a Kilian Pressima type press. The tableting is carried out with the addition of graphite. For this, the calcined precipitate obtained in step d) is mixed with the graphite, optionally compacted and/or granulated, and then tableted. The proportion of graphite in the mixture is in the range from 2.0% to 5.0% by weight, preferably from 2 to 4.5 %, more preferably from 2.0% to 4.0%, even more preferably from 3.0 to 4.0, most preferably from 3.1 to 4.0 % by weight, based on the total weight of the composition to be tableted.

When the distribution of at least Cu and Zn throughout the precipitate material is substantially homogenous, this results in a shaped catalyst body where at least Cu and Zn are simultaneously and substantially homogenously distributed in the areas originating from the precipitated material.

In one embodiment, a binder is added to the precipitate to be shaped. In principle, all compounds that increase the mechanical stability of the shaped body are suitable as binders. Suitable binders are aluminum oxide, such as pseudoboehmite, boehmite or corundum, silica, calcium aluminate, calcium silicate or clay minerals such as bentonite.

The binder is usually added to the mixture in such an amount that the content of binder in the mixture is within a range from 1% to 20% by weight, preferably within a range from 1% to 15% by weight, and particularly preferably within a range from 1% to 10% by weight, based on the total weight of the shaped body after loss on ignition.

The shaped catalyst body obtained in step e) is not subjected to any thermal treatment before being employed in the methanol synthesis process. In this context thermal treatment means any treatment at temperatures above 100 °C in air or under an oxygen containing atmosphere.

The shaped catalyst body obtainable by the process of the invention can in a further step be reduced before it is used in the catalytic reaction.

The reduction is here preferably carried out by heating the shaped catalyst body in a reducing atmosphere. The reducing atmosphere is especially 100 vol.% hydrogen or a mixture of hydrogen and inert gas. The reduction is carried out for example at a temperature within a range from 150°C to 450°C, preferably within a range from 160°C to 250°C, more preferably within a range from 180°C to 240°C. The reduction is carried out for example over a period of 1 hour to 20 days, preferably over a period of 2 hours to 120 hours, more preferably over a period of 24 to 48 hours

The present invention further provides a process for methanol synthesis by conversion of a CO₂ and H₂ containing gas mixture by use of the inventive shaped catalyst body. In one embodiment the so-called make-up gas, which is provided for the methanol synthesis and resulting for example from a preceding coal gasification process is a synthesis gas mixture, i.e. a gas containing CO₂, CO and H₂. The make-up gas usually substantially consists of from 2% by volume to 32% by volume of CO, from 1% by volume to 14% by volume of CO₂, from 0.1% by volume to 8% by volume of inert gases, e.g. N₂ and/or methane, with H₂ as balance. Such make-up gas prior to the reactor inlet can be combined with a recycle gas stream, which is separated from the product stream after the reactor and optionally being purged. Such combined gas feed is fed into the reactor as reactor inlet gas. In one embodiment the reactor inlet gas substantially consists of 5% to 16% by volume of CO, from 1% by volume to 7% by volume of CO₂, from 5% by volume to 15% by volume of inert gases, e.g. N₂ and/or methane, with H₂ as balancer. In another embodiment the reactor inlet gas substantially consists of 1% to 8% by volume of CO, from 1% by volume to 5% by volume of CO₂, from 10% by volume to 17% by volume of inert gases, e.g. N₂ and/or methane, with H₂ as balance. In another embodiment the reactor inlet gas substantially consists of 3% to 12% by volume of CO, from 5% by volume to 14% by volume of CO₂, from 8% by volume to 26% by volume of inert gases, e.g. N₂ and/or methane, with H₂ as balance. "Substantially" in the context of these embodiments means that the concerned gas composition contains less than 12000 ppmv, preferably less than 10000 ppmv, more preferably less than 5000 ppmv of components other than the mentioned ones.

In another embodiment a gas mixture consisting substantially of 17% by volume to 27% by volume CO₂, 73% by volume to 83% by volume H₂, up to 1% by volume of inert gases, e.g. N₂ and/or methane and less than 500 ppmv CO, is provided as make-up gas. Such make-up gas prior to the reactor inlet can be combined with a recycle gas stream, which is separated from the product stream after the reactor and optionally being purged. Such combined gas feed is fed into the reactor as reactor inlet gas. In one embodiment the reactor inlet gas substantially consists of 10% by volume to 14% by volume CO₂, 1% by volume to 5% by volume CO, 1% by volume to 10% by volume of inert gases, with H₂ as balance. "Substantially" in the context of these embodiments means that the concerned gas composition contains less than 12000 ppmv, preferably less than 10000 ppmv, more preferably less than 5000 ppmv of components other than the mentioned ones.

The methanol synthesis is usually carried out at a temperature in the range from 200°C to 300°C, preferably in the range from 210°C to 280°C, at a pressure in the range from 40 bar to 150 bar, preferably in the range from 60 bar to 100 bar, and a space velocity in the range from 2000 to 30 000 h⁻¹. The space velocity is defined as the ratio of the volume flow of synthesis gas to the spatial volume of the catalyst, e.g. of a catalyst bed, based on the time unit of 1 hour.

Figure 1 discloses the X-ray diffraction pattern of the Examples 8 and 11 as well as of monoclinic ZrO₂.

Figure 2 discloses the relative methanol yields over time on stream of Use Example 1.

Figure 3 discloses the relative methanol yields over time on stream of Use Example 3.

### Experimental section

### Determination of physical parameters

The physical parameters indicated in the present invention are, unless indicated otherwise, determined as described below:
Determination of the BET surface area: The BET surface area is determined by the nitrogen single-probe method in accordance with DIN 66132 on the pulverulent catalyst and on pellets as prepared according to the following examples.

Determination of the loss on drying: The determination of the loss on drying is carried out for a powder material. To determine the loss on drying of the pellets, these are milled beforehand to give powder. The sample to be determined is weighed out into a porcelain crucible which has previously been ignited at 600°C for 3 hours in a muffle furnace. The sample weighed into the ignited and tared porcelain crucible is subsequently thermally treated at 120 °C for 3 hours in a muffle furnace, transferred to a desiccator and cooled to room temperature. The cooled crucible is reweighed. The loss on drying at 120 °C is determined from the mass difference.

Determination of the loss on ignition: The determination of the loss on ignition is carried out for a powder material. To determine the loss on ignition of the pellets, these are milled beforehand to give powder. The sample to be determined is weighed out into a porcelain crucible which has previously been ignited at 600°C for 3 hours in a muffle furnace. The sample weighed into the ignited and tared porcelain crucible is subsequently thermally treated at 900 °C for 3 hours in a muffle furnace, transferred to a desiccator and cooled to room temperature. The cooled crucible is reweighed. The loss on ignition at 900 °C is determined from the mass difference.

Determination of the average side crush strength: The average side crush strength (SCS) of the shaped bodies is determined by using commercially available instruments, a ZWICK model Z1.0 is used for the samples in oxidic form, an ERWEKA TBH 425 TD is used for the reduced samples, in accordance with the instrument manufacturer's instructions. Typically, the pressures applied to the cylindrical wall of the pellets when rupture occurs is determined for a plurality of pellets (e.g. from 25 to 200, preferably from 50 to 150, for example 100 pellets). The arithmetic mean of the values obtained (in N) is calculated. The average side crush strength based on the cross-sectional area (in N/mm²) is given by normalization of the arithmetic mean obtained for the side crush strength on the basis of the arithmetic mean cross-sectional area. Cross-sectional area is determined by multiplying the tablet's height with the tablet's diameter. The average side crush strength based on the catalyst pellet mass (in N/g) is given by normalization of the arithmetic mean obtained for the side crush strength on the basis of the arithmetic mean catalyst pellet mass after loss on drying.

Determination of the pore volume of the pellets: The pore volume is determined by the mercury intrusion method in accordance with DIN 66133.

Elemental analysis of the catalysts has been carried out by an inductively coupled plasma emission spectroscopy (ICP).

The structure of any zirconium components within the samples was determined by X-ray diffractometry. This was done by analyzing the sample in a Bruker D4 Endeavor over a range from 5 to 90 °2Θ (step sequence 0.020 °2Θ, 1.5 seconds measurement time per step). The radiation used was CuKα1 radiation (wavelength 1.54060 Å, 40 kV, 35 mA). During the measurement, the sample stage was rotated about its axis at a speed of 30 rotations/min.

The content of fines below 1.7 mm was determined by providing a tube of 4 m length and 42 mm inner diameter, arranged vertically. 100 g of the tablets prepared according to the Examples were rapidly dumped into the tube and collected below the tube. The collected material was once again dumped two times into the tube. Afterwards the collected material was sieved with a sieve of 1.7 mesh. The amount of fines below 1.7 mm diameter was weighted, and the relative weight percentage was calculated based on the 100 g catalyst material used for the test.

### Examples

### Example 1

29.3 kg of a sodium carbonate solution (sodium carbonate content 14.7 wt.%) were provided. In parallel, 12.4 kg of an aqueous copper nitrate solution (copper content 11.3 wt.%), a solution prepared by dispersing 713 g ZnO in 3.0 kg of a 34 wt.% nitric acid and 2.6 kg of a sodium aluminate solution (4 wt.% NaAlO₂), followed by addition of 404 g of a 68 wt.% nitric acid were provided. The Al-containing solution and the zinc containing solution were combined. At the same time, 391 g zirconium carbonate (zirconium content 29.6 wt.%) was dispersed in 1.5 kg of deionized water during stirring. After that 100 g of 68 wt.% nitric acid was added, heated for 2 hours at 50 °C, and stirred until dissolution. Finally, the aluminum and zinc containing solution and the zirconium containing solution were transferred to the copper nitrate solution. The resulting solution was combined with additional deionized water to result in a total volume of 20 L. The metal nitrate solution and the sodium carbonate solution were both preheated to 65 °C before the precipitation step.

For the precipitation step, in a precipitation vessel 82.5 g of pseudoboehmite (38.1 wt. % of aluminum) were dispersed in 6 L of deionized water at room temperature. After that the metal nitrate solution and the sodium carbonate solution were pumped simultaneously into the precipitation vessel through a mixing nozzle. The pH was controlled in the range of 6.5±0.1. Once precipitation was finalized, the suspension was additionally aged in the aging vessel for 60 minutes at 65 °C.

After aging, the suspension was filtered and the filter cake washed with deionized water until the conductivity was <200 µS/cm. The filter cake was resuspended with deionized water to result in a suspension with 10 % wt.% solid amount and afterwards spray dried at 275-280 °C inlet temperature and 105-115°C outlet temperature. The resulting powder was calcined in a rotary calciner at a temperature of 470 °C.

The obtained powder was pressed to catalyst tablets with a Kilian Pressima rotary tablet press. Before the tableting step the powder was homogenously mixed with graphite in such an amount, that the final catalyst tablets contained 1.75 wt.% graphite, based on the tablet weight. The tablet size was 6x4 mm and the average side crush strength (SCS) was 200 N.

### Example 2

27 L of a 1.5 M sodium carbonate solution was prepared by dissolving sodium carbonate in water. 20 L of a metal containing aqueous medium (metal molar concentration 1.5 M with molar ratio of the metals in the solution being Cu:Zn:Al:Zr:Hf = 62.1:29.8:3.1:3.4:1.6) was prepared by mixing corresponding amounts of metal precursors of copper, zinc, aluminum, zirconium and hafnium in deionized water containing nitric acid required for dissolution of the metal precursors. The metal containing mixture was heated to 50°C under controlled stirring for 2 hours until complete dissolution was achieved. The pH value after dissolution was in the range 2-2.4.

For the precipitation step, in a precipitation vessel 74.8 g of pseudo boehmite as used for Example 1 was dispersed in 6 L of deionized water at room temperature. After that the metal nitrate solution and the sodium carbonate solution were pumped simultaneously into the precipitation vessel. The pH was controlled in the range of 6.5±0.5. Once precipitation was completed, the suspension was additionally aged in the aging vessel for 60 minutes at 65 °C.

After aging, the suspension was filtered and the filter cake washed with deionized water until the conductivity was <200 µS/cm. The filter cake was resuspended with deionized water to result in a suspension with 10 w.% solid amount and afterwards spray dried at 275-280 °C inlet temperature and 105-115°C outlet temperature. The resulting powder was calcined in a rotary calciner at a temperature of 470 °C.

The obtained powder was tableted with a Kilian Pressima rotary tablet press, after the powder was homogenously mixed with graphite in such an amount, that the final catalyst tablets contained 3.5 wt.% graphite, based on the tablet weight after loss of drying. The tablet size was 5x3 mm and the average side crush strength (SCS) was 90 N.

### Example 3

The catalyst of Example 3 was prepared according to the preparation procedure of Example 2, but the molar ratio of metals in the metal nitrates solution corresponds to Cu:Zn:Al:Zr:Hf = 62.2:27:3.1:7.5:0.1, the mass of pseudo boehmite dispersed in the precipitation vessel equals 70.2 g and the final catalyst tablets contained 1.7 wt.% graphite, based on the tablet weight after loss of drying. The tablet size was 6x4 mm and the average side crush strength (SCS) was 200 N.

### Example 4

The catalyst of Example 4 was prepared according to the preparation procedure of Example 2, but molar ratio of metals in the metal nitrates solution corresponds to Cu:Zn:Al:Zr:Hf = 64.9:27.9:3.1:4.1:0.05, the mass of pseudo boehmite dispersed in the precipitation vessel equals 68.8 g and the final catalyst tablets contained 1.7 wt.% graphite, based on the tablet weight after loss of drying. The tablet size was 6x4 mm.

### Example 5

The catalyst of Example 5 was prepared according to the preparation procedure of Example 2, but the molar ratio of metals in the metal nitrates solution corresponds to Cu:Zn:Al = 59.4:25.6:15, no pseudo boehmite was dispersed in the precipitation vessel and the final catalyst tablets contained 1.7 wt.% graphite, based on the tablet weight after loss of drying. The tablet size was 6x4 mm.

### Example 6

The catalyst of Example 6 was prepared according to the preparation procedure of Example 2, but the molar ratio of metals in the metal nitrates solution corresponds to Cu:Zn:Al = 66.9:30.2:2.9 and the mass of pseudo boehmite dispersed in the precipitation vessel equals 206 g. The tablet size was 6x4 mm.

### Example 7

The catalyst of Example 7 was prepared according to the preparation procedure of Example 2, but the molar ratio of metals in the metal nitrates solution corresponds to Cu:Zn:Al:Zr:Hf = 64.7:28.1:3.1:3.8:0.23 and the final catalyst tablets contained 1.7 wt.% graphite, based on the tablet weight after loss of drying.

### Example 8

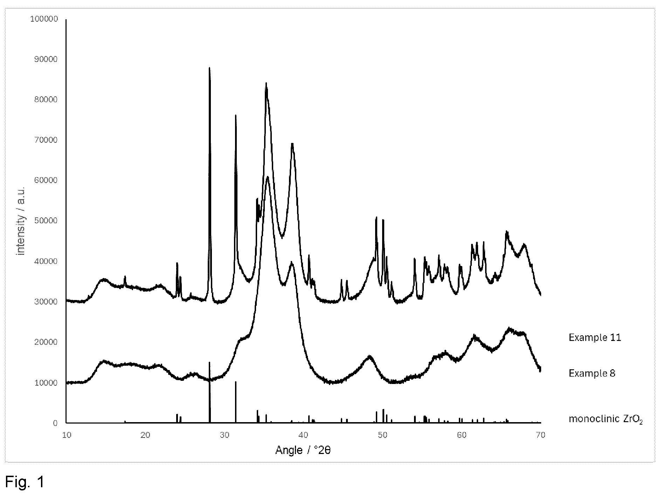

The catalyst of Example 8 was prepared according to the preparation procedure of Example 2, but the molar ratio of metals in the metal nitrates solution corresponds to Cu:Zn:Al:Zr:Hf = 64.8:28.1:3.1:3.9:0.04. The tablet size was 6x4 mm. Fig. 1 shows the X-ray diffraction pattern of the calcined powder.

### Example 9

The catalyst of Example 9 was prepared according to the preparation procedure of Example 5, but the final catalyst tablets contained 3.7 wt.% graphite, based on the tablet weight after loss of drying. The tablet size was 6x4 mm.

### Example 10

The catalyst of Example 10 was prepared according to the preparation procedure of Example 5, but the final catalyst tablets contained 5 wt.% graphite, based on the tablet weight after loss of drying. The tablet size was 6x4 mm.

### Example 11

The catalyst of Example 11 was prepared according to the preparation procedure of Example 2, but the molar ratio of metals in the metal nitrates solution corresponds to Cu:Zn:Al = 67.3:29.3:3.4 and 80.9 g of pseudo boehmite as well as 302 g of ZrO₂ were dispersed in the precipitation vessel. Fig. 1 shows the X-ray diffraction pattern of the calcined powder.

### Example 12

The catalyst of Example 12 was prepared according to the preparation procedure of Example 2, but the molar ratio of metals in the metal nitrates solution corresponds to Cu:Zn:Al:Zr = 64.8:28.1:3.1:3.9 and 84.9 g of TiO₂ was dispersed in the precipitation vessel. The Ti content, calculated as TiO₂, of the tablet material was 3.3 wt.%.

### Example 13

The catalyst of Example 13 was prepared according to the preparation procedure of Example 1, but instead 753 g ZnO, 5.4 kg of the sodium aluminate solution (4 wt.% NaAlO₂), 161 g zirconium carbonate (zirconium content 29.6 wt.%), 66.8 g of pseudo boehmite, and correspondingly adjusted amount of nitric acid were used during the preparation.

The chemical and physical properties of the prepared catalysts are summarized in Table 1, whereby the contents of Cu, Zn, Al, Zr, Hf and Ti refer to the contents of the corresponding oxides. For all these elements the values are based on the catalyst weight after loss of ignition. The graphite values are based on the catalyst weight after loss of drying.

**Table 1: Overview of catalysts according to Examples 1 to 13**

| Example | Addition of pseudo boehmite | Cu content, calculated as CuO [wt.%] | Zn content, calculated as ZnO [wt.%] | Al content, calculated as Al₂O₃ [wt.%] | Zr content, calculated as ZrO₂ [wt.%] | Hf content, calculated as HfO₂ [wt.%] | Graphite content [wt.%] | SCS [N] | SCS after reduction [N] | Fines below 1.7 mm |
|---|---|---|---|---|---|---|---|---|---|---|
| Example 1 | yes | 61.6 | 28.4 | 4.0 | 6.0 | - | 1.75 | 200 | not determined | not determined |
| Example 2 | yes | 57.5 | 28.7 | 4.3 | 5.4 | 4.1 | 3.5 | 90 | not determined | not determined |
| Example 3 | yes | 57.9 | 27.1 | 3.9 | 10.9 | 0.2 | 1.7 | 204 | 63 | not determined |
| Example 4 | yes | 61.6 | 28.4 | 3.9 | 6.0 | 0.1 | 1.7 | 201 | 42 | 1.3 |
| Example 5 | no | 62.3 | 27.8 | 9.9 | - | - | 1.7 | 185 | 51 | 1.7 |
| Example 6 | yes | 63.3 | 28.5 | 8.2 | - | - | 1.7 | 187 | 47 | 2.6 |
| Example 7 | yes | 61.1 | 28.5 | 3.9 | 5.9 | 0.6 | 3.5 | 74 | not determined. | not determined |
| Example 8 | yes | 61.6 | 28.3 | 4.1 | 5.9 | 0.1 | 3.5 | 205 | 54 | 1.2 |
| Example 9 | no | 62.3 | 27.8 | 9.9 | - | - | 3.7 | 249 | 56 | not determined |
| Example 10 | no | 62.3 | 27.8 | 9.9 | - | - | 5 | 237 | 45 | not determined |
| Example 11 | yes | 59.4 | 26.3 | 3.8 | 10.5 | - | 3.5 | 44 | 20 | not determined |
| Example 12 | no, TiO2 instead | 62.0 | 27.1 | 1.9 | 5.7 | - | 3.5 | 59 | 14 | not determined |
| Example 13 | yes | 64.6 | 27.3 | 5.7 | 2.4 | - | 1.7 | 200 | 38 | not determined |

### Use Example 1

The catalysts of Example 4, Example 5 and Example 6 were utilized in a catalytic process for methanol synthesis.

The tablets of the catalyst samples to be tested were crushed, sieved to a fraction of 2.5 - 3.5 mm and diluted (1:1 volume based) with alumina spheres having diameters of 2 - 4 mm before loading. A stainless steel reactor was filled with 11.25 g catalyst. The catalyst materials were reduced by heating in a gas mixture of 2.0 % H₂/N₂ to 110 °C (at 0.5 °C · min⁻¹) followed by heating to 250°C (at 0.133 °C · min⁻¹), followed by holding the material at 250°C for 1 hour in 2.0 % H₂/N₂ and finally holding for 2 hours in pure H₂. Heating temperature of all Use Examples was controlled by measuring the temperature at the rear end of the downstream catalyst bed.

Finally the feed gas stream was switched to nitrogen and pressurized to 60 bar, and after that a synthesis gas mixture consisting of 14.2 Vol.% CO₂, 70.0 Vol.% H₂, 1.6 Vol.% CO, 14.2 Vol.% inerts was fed. The pressure was 60 bar and the flow rate was 300 NL/h.

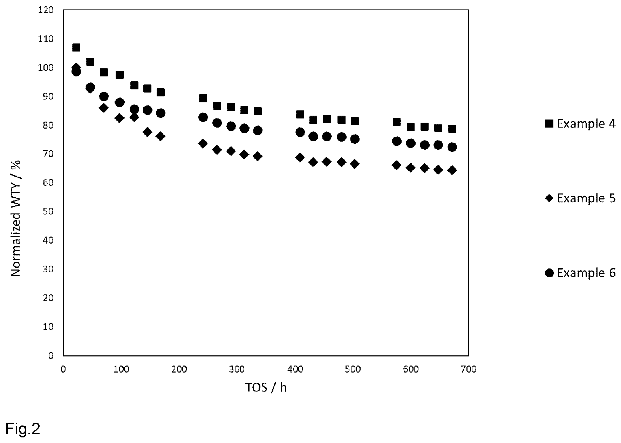

Figure 2 discloses the relative methanol yields over time on stream. The weight time yields are shown relatively to the catalyst of Example 5.

### Use Example 2

The catalysts of Example 2 to 13 were utilized in a further catalytic process for methanol synthesis under different process conditions as in Use Example 1.

A stainless steel microreactor was filled with 200 mg of catalyst material (sieve fraction 250-355 µm). The catalyst materials were reduced by heating in a gas mixture of 2.0 % H₂/N₂ to 110 °C (at 0.5 °C · min⁻¹) followed by heating to 250°C (at 0.133 °C · min⁻¹), followed by holding the material at 250°C for 1 hour in 2.0 % H₂/N₂ and finally holding for 2 hours in pure H₂.

Finally, the feed gas stream was switched to nitrogen and pressurized to 60 bar, and after that a synthesis gas mixture consisting of 14.2 Vol.% CO₂, 70.0 Vol.% H₂, 1.6 Vol.% CO, 14.2 Vol.% inerts was fed. The pressure was 60 bar and the flow rate was 300 NL/h.

For the first run, the initial methanol WTY was determined and after 70 h the temperature was decreased to 210 °C and kept at this temperature for 20 h, whereby the methanol WTY was again determined. Afterwards the temperature was increased to 270 °C for 150 h to accelerate ageing of the catalyst samples. Afterwards in the second run, temperature was again set to 250 °C, kept for 20 h at this temperature and the WTY after the ageing step at 270 °C was determined. Then temperature was decreased to 210 °C and kept at this temperature for 20 h and also at this temperature the methanol WTY after the ageing step at 270 °C was determined.

Table 2 discloses the initial relative methanol WTY at 250 °C and subsequently at 210 °C, as well as WTY after accelerated ageing, at 250 °C and 210 °C, respectively. The weight time yields are shown relatively to the catalyst of Example 5.

**Table 2: Relative weight time yields of the catalysts according to Example 2 to Example 13.**

| Catalyst | 1st run | | 2nd run | |
|---|---|---|---|---|
| | Relative WTY at 250 °C [%] | Relative WTY at 210 °C [%] | Relative WTY at 250 °C [%] | Relative WTY at 210 °C [%] |
| Example 2 | 106 | 106 | 112 | 114 |
| Example 3 | 115 | 119 | 119 | 122 |
| Example 4 | 115 | 125 | 116 | 125 |
| Example 5 | 100 | 100 | 100 | 100 |
| Example 6 | 107 | 110 | 111 | 114 |
| Example 7 | 106 | 106 | 109 | 105 |
| Example 8 | 113 | 120 | 118 | 122 |
| Example 9 | 98 | 96 | | |
| Example 10 | 100 | 93 | | |
| Example 11 | 106 | 117 | 112 | 122 |
| Example 12 | 115 | 119 | 124 | 124 |
| Example 13 | 105 | 112 | 103 | 112 |

### Use Example 3

The catalysts of Example 4, Example 5 and Example 6 were utilized in a further catalytic process for methanol synthesis under different process conditions as in Use Example 1.

The tablets of the catalyst samples to be tested were crushed, sieved to a fraction of 2.5 - 3.5 mm and diluted (1:1 volume based) with alumina spheres having diameters of 2 - 4 mm before loading. A stainless steel reactor was filled with 11.25 g catalyst. The catalyst materials were reduced by heating in a gas mixture of 2.0 % H₂/N₂ to 110 °C (at 0.5 °C · min⁻¹) followed by heating to 250°C (at 0.133 °C · min⁻¹), followed by holding the material at 250°C for 1 hour in 2.0 % H₂/N₂ and finally holding for 2 hours in pure H₂.

Finally, the feed gas stream was switched to nitrogen and pressurized to 60 bar, and after that a gas mixture consisting of 20.0 Vol.% CO₂, 60.0 Vol.% H₂, 20.0 Vol.% inerts was fed. The pressure was 60 bar and the flow rate was 300 NL/h.

The test was run under these conditions for 600 h, and WTY was determined after several periods. Then, temperature was decreased to 210 °C and WTY was once again determined after several periods. The overall test run lasted 700 h.

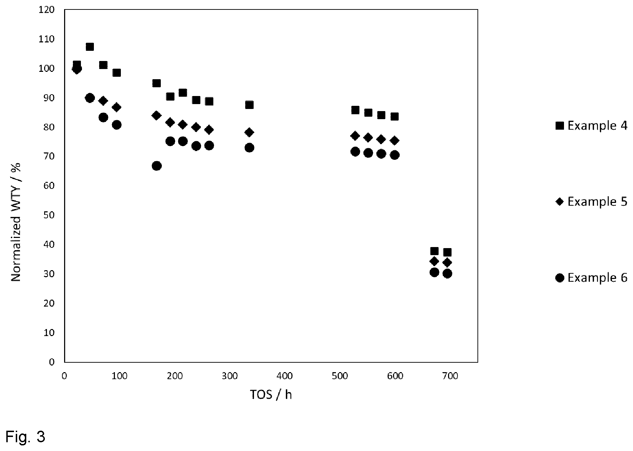

Figure 3 discloses the relative methanol yields over time on stream. The weight time yields are shown relatively to the catalyst of Example 6.

## Claims

1. A shaped catalyst body for methanol synthesis containing Cu, Zn and Al, **characterized in that** the shaped catalyst body contains zirconium, calculated as ZrO₂, in a proportion by weight in the range from 1% to 10%, preferably from 2 to 9 % by weight, more preferably from 3 to 8 % by weight, most preferably from 4 to 7 % by weight, based on the total weight of the shaped catalyst body after loss on ignition, and graphite in a proportion by weight in the range from 2 to 5 %, preferably from 2 to 4.5 %, more preferably from 2 to 4 %, even more preferably from 3 to 4 %, most preferably from 3.1 to 4.0 %, based on the total weight of the shaped catalyst body after loss on drying.

2. The shaped catalyst body according to claim 1, wherein the shaped catalyst body further contains a metal with oxidation state +4, calculated as its oxide, preferably hafnium, in a proportion by weight in the range from 0.05% to 10%, preferably from 0.08 to 4.0 % by weight, more preferably from 0.1 to 4.0, based on the total weight of the shaped catalyst body after loss on ignition.

3. The shaped catalyst body according to any of claim 1 or 2, wherein the BET surface area of the shaped catalyst body, as determined by nitrogen physisorption, is in the range from 75 to 150 m2/g, preferably from 80 to 140 m2/g, more preferably from 90 to 140 m2/g, even more preferably from 90 to 130 m2/g.

4. The shaped catalyst body according to any of claim 1 to 3, wherein the zirconium is present in its oxidic form and X-ray amorphous.

5. The shaped catalyst body according to any of claim 1 to 4, further comprising either Na in a proportion by weight below 1%, or K in a proportion by weight below 1%, or Cs in a proportion by weight below 1%, or Fe in a proportion by weight below 0.1%, or Ni in a proportion by weight below 0.1%, or Cr in a proportion by weight below 0.1%, or Mn in a proportion by weight below 0.1%, or Ca in a proportion by weight below 5%, or Mg in a proportion by weight below 5%, or Bi in a proportion by weight below 0.1%, or Cl in a proportion by weight below 1%, or S in a proportion by weight below 0.1%, or As in a proportion by weight below 0.1%, or any combination thereof, based on the total weight of the shaped catalyst body after loss on ignition.

6. The shaped catalyst body according to any of claim 1 to 5, present in form of an extrudate or tablet or pellet.

7. The shaped catalyst body according to any of claim 1 to 5, present in form of a tablet and **characterized by** an average side crush strength from 80 to 300 N.

8. The shaped catalyst body of claim 7, wherein the diameter of the tablets is in the range from 2 to 8 mm, preferably from 3 to 7 mm, particularly preferably from 4.7 to 6.2 mm, and the height of the tablets is in the range from 2 to 6 mm, preferably from 2.5 to 5 mm, particularly preferably in the range from 2.8 to 4.2 mm.

9. The shaped catalyst body of any of claim 7 or 8, **characterized by** an average side crush strength after reduction from 20 to 80 N, preferably 30 to 60 N, more preferably 35 to 60 N.

10. The shaped catalyst body of any of claim 1 to 9, wherein the Cu/Zn atomic is from 15:85 to 85:15, preferably from 60:40 to 75:25 and the Zn/Al atomic ratio is from 60:40 to 80:20, preferably from 70:30 to 80:20.

11. The shaped catalyst body of any of claim 1 to 10, wherein the weight percentage of copper, calculated as CuO, is in the range from 40 to 70 %, preferably from 50 to 65 %, more preferably from 60 to 65 %, the weight percentage of zinc, calculated as ZnO, is in the range from 17 to 35 %, preferably from 20 to 30 %, more preferably from 25 to 30 %, and the weight percentage of aluminum, calculated as Al₂O₃, is in the range from 1 to 10 %, preferably from 2 to 8 %, more preferably from 3 to 6 %, all based on the total weight of the shaped catalyst body after loss on ignition.

12. A method for production of the shaped catalyst body according to any of claims 1 to 11, said method comprising the following steps:
a) combining (i) at least one aqueous medium A comprising copper compounds, zinc compounds, and optionally further transition metal compounds and (ii) at least one aqueous alkaline medium B to form a precipitate, wherein one/or more dissolved and/or dispersed aluminum compound and one or more zirconium compound is present in medium A and/or medium B and/or introduced to the mixture of medium A and B,
b) separating off the precipitate, optionally washing the precipitate,
c) drying the precipitate to obtain a dried precipitate,
d) calcining the dried precipitate from step c) at a temperature of between 300 and 800°C for a period of between 15 min and 4 h.
e) shaping a mixture of the calcined precipitate from step d) and graphite to obtain a shaped body.

13. The method according to claim 12, wherein medium A and medium B are jointly fed into a common precipitation vessel.

14. The method according to any of claim 12 or 13, wherein the pH during the precipitation in step a) is within a range from 6.0 to 8.0, preferably within a range from 6.0 to 7.5, more preferably within a range from 6.0 to 7.0.

15. A process for methanol synthesis by use of the shaped catalyst body according to any of claim 1 to 11 or by use of a catalyst produced according to any of claim 12 to 14.

16. The process according to claim 15, wherein a make-up gas, substantially consisting of from 2% by volume to 32% by volume of CO, from 1% by volume to 14% by volume of CO₂, from 0.1% by volume to 8% by volume of inert gases, e.g. N₂ and/or methane, with H₂ as balance is used.

17. The process according to claim 15, wherein a make-up gas, substantially consisting of 17% by volume to 27% by volume CO₂, 73% by volume to 83% by volume H₂, up to 1% by volume of inert gases, e.g. N₂ and/or methane and less than 500 ppmv CO is used.

18. The process according to any of claim 15 to 17, wherein the make-up gas is combined with a recycle gas stream.

19. The process according to any of claims 13 to 18, wherein the temperature is the range from 200°C to 300°C, preferably in the range from 210°C to 280°C, the pressure is in the range from 40 bar to 150 bar, preferably in the range from 60 bar to 100 bar, and the space velocity is in the range from 2000 to 30000 h⁻¹.
